(19) Europäisches Patentamt
European Patent Office
Office européen des brevets

(11) Publication number: **0 031 120**
**B1**

(12) **EUROPEAN PATENT SPECIFICATION**

(45) Date of publication of patent specification: **01.08.84**

(21) Application number: **80107914.6**

(22) Date of filing: **15.12.80**

(51) Int. Cl.³: **C 07 D 471/04**
//(C07D471/04, 239/00, 221/00)

(54) **A process for the production of 2-substituted 6-alkoxycarbonyl-8-ethyl-5-oxo-5,8-dihydro-pyrido(2,3-d)-pyrimidines and of corresponding free acids.**

(30) Priority: **21.12.79 IT 2832279**

(43) Date of publication of application:
**01.07.81 Bulletin 81/26**

(45) Publication of the grant of the patent:
**01.08.84 Bulletin 84/31**

(84) Designated Contracting States:
**AT BE CH DE FR GB LI NL SE**

(56) References cited:
**CA - A - 1 066 701**
**GB - A - 1 406 381**

(73) Proprietor: **Unibios S.p.A.**
**Via Silvio Pellico, n.3**
**I-28069 Trecate (Novara) (IT)**

(73) Proprietor: **Prodotti Chimici Alimentari S.p.A.**
**Via Novi, 58/A**
**Basaluzzo (Alessandria) (IT)**

(72) Inventor: **Chiodoni, Ugo**
**Corso Sempione, 104**
**I-20100 Milano (IT)**
Inventor: **Parenti, Massimo**
**Corso Italia, 25**
**Novi Ligure Alessandria (IT)**
Inventor: **Spinelli, Silvano**
**Via Zanella 66**
**I-20100 Milano (IT)**

(74) Representative: **Dragotti, Gianfranco et al,**
**SAIC BREVETTI S.a.s. di Ing. G. Dragotti & C. Via Spontini, 1**
**I-20131 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 031 120 B1

**Description**

The present invention relates to a novel process for the production of 2-substituted 6-alkoxycarbonyl-8-ethyl-5-oxo-5,8-dihydro-pyrido[2,3-d]pyrimidines and their free acids, having the following general formula A;

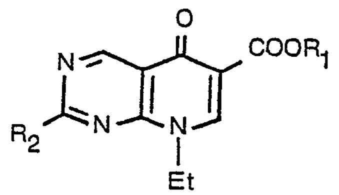

(A)

wherein

$R_1 = H$, $CH_3$ or $C_2H_5$ and
$R_2 = Cl$, $CH_3O$, $C_2H_5O$, $CH_3S$ or $CH_3SO_2$.

These compounds are used in the condensation with secondary amines for the preparation of compounds having a relevant bacteriostatic activity, such as the pyromidic and the pipemidic acids.

As a matter of fact, the compounds of the general formula A constitute, as it is known from the prior art, useful intermediates for the production of the aforesaid acids and the several processes (e.g. U.K. Patent No. 1,406,381) as used up to date for their production, consisted in reacting the corresponding 5,6,7,8-tetrahydroderivatives with a halogen, preferably bromine, in the elimination of a molecule of the corresponding hydrogen halide by means of a tertiary amino base, preferably triethylamine.

According to further suggested processes, (e.g. Canadian Patent No. 1,066,710), the use of quinone reactants, such as chloranile is foreseen, without any advantage from the point of view of the industrial process.

This reference does also disclose a number of ways to get the desired dehydrogenation. More particularly, the proposed dehydrogenation steps involve the intermediate 6-alkoxy-carbonyl-8-ethyl-5-oxo-5,6,7,8-tetrahydro-pyrido[2,3-d]pyrimidine, and contemplate:

a) direct heating without solvent, at a temperature higher than the meeting point;
b) heating in a solvent, without dehydrogenating agent;
c) dehydrogenation with 10% palladium carbon.

However, in the case (a) and (b) no dehydrogenation takes place at all, whereas the dehydrogenation occurs with the starting compounds of the Canadian patent but not with different compounds 5-oxo-6-carbalkoxy-5,6,7,8-tetrahydro-pyrido[2,3-d]pyrimidines.

It has been now found that the 5,6,7,8-tetrahydroderivatives can be dehydrogenated to the corresponding compounds of general formula A by means of a group of oxidizing compounds, which are less expensive, give very high yields and require for their industrial use a less sophisticated and safer technology with respect to that needed for the use of bromine or of other halogens.

These oxidizing compounds, which act according to a mechanism different, from that of the halogens, are for instance alkaline dichromates, alkaline permanganates or nitric acid.

Their action takes directly place in solution or suspension, which is preferably neutral or made acidic by the addition of acetic acid and aqueous acetic acid, or of diluted mineral acids, directly leading to desired product without using other reactants, as it was necessary in the case of using bromine or sulfuryl chloride which then required triethylamine as the acceptor of hydrogen halide in a subsequent step. Thus, according to the process of the present invention, a compound having the general formula B:

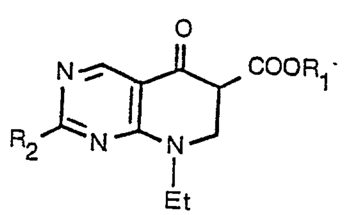

(B)

$R_1 = H$, $CH_3$ or $C_2H_5$ and
$R_2 = Cl$, $CH_3O$, $C_2H_5O$, $CH_3S$ or $CH_3SO_2$

is reacted with an oxidizing compound, preferably selected from dichromates and permanganates of

alkali metals or nitric acid, the reaction being carried out in hot conditions until the conversion is completed.

More particularly, the 6-carbethoxy-8-ethyl-2-ethoxy-5-oxo-5,8-dihydro-pyrido[2,3-d]pyrimidine I has been found to be especially useful for the preparation with high yields of the above mentioned acids.

It is thus prepared, according to the present invention, from 6-ethoxycarbonyl-8-ethyl-2-ethoxy-5-oxo-5,6,7,8-tetrahydro-pyrido[2,3-d]pyrimidine II, according to the following scheme:

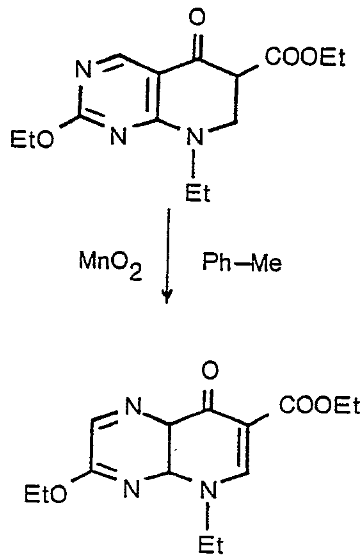


(II)

(I)

In turn, the compound (II) is prepared in two steps, according to the prior art, starting from 2-ethoxy-4-chloro-5-carbethoxy-pyrimidine, which is condensed with ethyl $\beta$-ethylamino-propionate in the presence of an acid acceptor and then cyclized in the presence of alkaline alcoholates. The following examples illustrate the invention, without having limiting meaning.

### Example 1

A 1 liter flask is charged with 250 g of water and 160 mls of 12.5% nitric acid.

The solution is heated to 75—80°C and at this temperature 50 g of 6-carbethoxy-2-methylthio-5-oxo-8-ethyl-5,6,7,8-tetrahydro-pyrido[2,3-d]pyrimidine are added in portions.

The oxidation reaction begins after 3—4 portions of the compound to be oxidized have been added.

The internal temperature of the flask is maintained at 75 to 80°C, by slightly heating. Once the addition of intermediate compound is completed, after about 30 minutes, the suspension is brought to about 90—95°C and then maintained at that temperature for 30 minutes.

Thereafter the reaction mixture is cooled to the room temperature. The resulting white product is decanted. The acid mother liquor is removed by siphoning and 150 mls of water are added to the flask the reaction mixture is stirred for 5 minutes and then decanted again. The supernatant water is removed by siphoning and then 90 mls of 5 M potassium hydroxyde and 150 mls of water are added, whereby all the product is dissolved, 50 mls of chloroform are added to this solution.

The reaction mixture is stirred for 5 minutes and then the two phases are allowed to separate. The chloroform phase is removed and the washing with chloroform (50 mls) is repeated. The reaction mixture is maintained under stirring for 5 minutes, the two phases are allowed to separate, and the chloroform phase is removed. By adding 40 mls of diluted acid acetic acid (1:1) a precipitate is obtained from the aqueous phase up to pH 5—5,5.

The product is filtered, washed with water and then with ethanol.

There are obtained 38.5 g of 2-methylthio-5-oxo-8-ethyl-5,8-dihydro-pyrido[2,3-d]pyrimidin-6-carboxylic acid, (with a yield of 86% with respect to the compound to be oxidized). M.p.: 254—266°C.

### Example 2

Preparation of 6-ethoxycarbonyl-8-ethyl-2-ethoxy-5-oxo-5,8-dihydro-pyrido[2,3-d]pyrimidine.

To a solution of 5.8 g of the corresponding tetrahydro derivative in 30 mls of acetic acid, heated to 50°C, 8 g of sodium bichromate, as a solution in 35 mls of water, are dropwise added. The addition takes place in 30 minutes. The deep green solution is furthermore stirred for one and half hours at 50°C and then diluted with 3 times the volume thereof with cold water. The resulting suspension is neutralized with sodium hydroxyde (pH 7) and extracted with $CHCl_3$.

The organic phase, after drying on $Na_2SO_4$ and concentration to dry residue, gives place to a white precipitate which is crystallized from isopropanol (2:1); there are obtained 4.5 g of product (m.p.: 144°C). Yield: 78.1%. From the preceding examples it is clear that, by means of the process of the present invention, it is possible, once the oxidizing agent and the solvent are carefully selected, to prepare the compounds of general formula A either as esters or as free acids: more particularly, the former are obtained when the oxidizing agent is an alkali dichromate and the solvent is acetic acid (aqueous or diluted), or a mineral acid (also in diluted form), such as for instance sulphuric acid. It is important when the compound A serves for further reactions, in which the free acid form might be an obstacle with respect to an easy performance of the next step.

**Claims**

1. Process for the preparation of 2-substituted 6-alkoxycarbonyl-8-ethyl-5-oxo-5,8-dihydro-pyrido[2,3-d]pyrimidines, of general formula A:

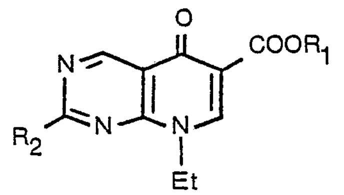

(A)

in which

$R_1$ is H, $CH_3$ or $C_2H_5$ and
$R_2$ is Cl, $CH_3O$, $C_2H_5O$, $CH_3S$ or $CH_3SO_2$

wherein the corresponding tetrahydro derivatives are dehydrogenated under hot conditions, characterized in that the tetrahydroderivative is treated with dehydrogenating agent selected from alkali dichromates, alkali permanganate or nitric acid, the solvent being selected from acetic acid, aqueous acetic acid or a dilute mineral acid.

2. Process according to claim 1, characterized in that 6-carboethoxy-8-ethyl-2-ethoxy-5-oxo-5,8-dihydro-pyrido[2,3-d]pyrimidine, of the general formula A wherein $R_1$ is $C_2H_5$ and $R_2$ is $C_2H_5O$, is prepared by starting from the corresponding tetrahydro derivative.

3. Process according to claim 1, characterized in that an alkaline dichromate is used in the dehydrogenation and the reaction mixture is maintained at a temperature of between 40°C and the boiling temperature of the solvent.

**Patentansprüche**

1. Verfahren zur Herstellung von 2-substituierten 6-Alkoxycarbonyl-8-äthyl-5-oxo-5,8-dihydro-pyrido[2,3-d]pyridinen der allgemeinen Forme A:

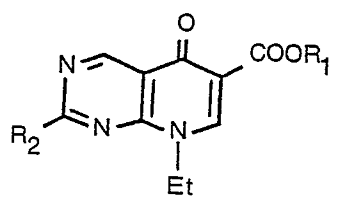

(A)

worin

$R_1 =$ H, $CH_3$ oder $C_2H_5$ und
$R_2 =$ Cl, $CH_3O$, $C_2H_5O$, $CH_3S$ oder $CH_3SO_2$

sind, bei dem die entsprechenden Tetrahydoderivative unter Wärme dehydrogeniert sind, dadurch gekennzeichnet dass das Tetrahydroderivat mit einem Dehydrogenationsmittel behandelt wird, welches unter Alkalidichromaten, Alkalipermanganat oder Salpetersäure ausgewählt ist, wobei das Lösungsmittel unter Essigsäure, wässriger Essigsäure oder verdünnter Mineralsäure ausgewählt wird.

2. Verfahren nach Anspruch 1, dadurch gekennzeichnet, dass das 6-Carboäthoxy-8-äthyl-2-äthoxy-5-oxo-5,8-dihydro-pyrido[2,3-d]pyridin der allgemeinen Formel A, worin $R_1$ $C_2H_5$ und $R_2$ $C_2H_5O$ sind, dadurch hergestellt wird, dass von dem entsprechenden Tetrahydroderivat ausgegangen wird.

3. Verfahren nach Anspruch 1, dadurch gekennzeichnet, dass bei der Dehydrogenation ein alkalinisches Dichromat verwendet wird und dass das Reaktionsgemisch auf einer Temperatur zwischen 40°C und der Siedetemperatur des Lösungsmittels gehalten wird.

## Revendications

1. Procédé de préparations de 6-alkoxycarbonyl-8-éthyl-5-oxo-5,8-dihydropyrido[2,3-d]-pyrimidines substituées en position 2 de formule générale A:

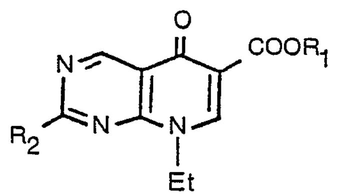

(A)

dans laquelle

$R_1$ est H, $CH_3$ ou $C_2H_5$ et
$R_2$ est Cl, $CH_3O$, $C_2H_5O$, $CH_3S$ ou $CH_3SO_2$

ou les tétra-hydro dérivés correspondants sont déshydrogénés à chaud caractérisé en ce que le tétra-hydro dérivé est traité avec un agent déshydrogénant choisi parmi les bichromates alcalines, les permanganats alcalins et l'acide nitrique, le solvant étant choisi parmi acide acetique, acide acetique aqueux ou un acide mineral dilué.

2. Procédé selon la revendication 1, caractérisé en ce que 6-carboéthoxy-8-éthyl-2-éthoxy-5-oxo-5,8-dihydro-pyrido[2,3-d]-pyrimidines de la formule générale A dans laquelle $R_1$ est $C_2H_5$ et $R_2$ est $C_2H_5O$, est préparé à partir du tétra-hydro dérivé correspondant.

3. Procédé selon la revendication 1, caractérisé en ce que dans la déshydrogenation on utilise un bichromate alcalin et le mélange de réaction est maintenu à une température entre 40°C et la température d'ébullition su solvant.